# EUROPEAN PATENT APPLICATION

(11) **EP 4 548 944 A1**
(43) Date of publication of application: **07.05.2025**
(21) Application number: 23871901.7
(22) Date of filing: 12.09.2023
(51) Int. Cl.: A61M 1/16, A61M 1/14, A61M 1/36

(54) **COMPUTER PROGRAM, EXTRACORPOREAL CIRCULATION SYSTEM, OBSERVATION DEVICE, AND INFORMATION PROCESSING METHOD**

(30) Priority: 29.09.2022 JP 2022156626
(71) Applicant: Terumo Kabushiki Kaisha, Tokyo 151-0072 (JP)
(72) Inventor: INOUE, Junji, Ashigarakami-gun, Kanagawa 259-0151 (JP); TONG, Tong, Ashigarakami-gun, Kanagawa 259-0151 (JP); SAITO, Takashi, Ashigarakami-gun, Kanagawa 259-0151 (JP)
(74) Representative: KIPA AB
(86) International application number: PCT/JP2023/033226
(87) International publication number: WO 2024/070668

(57) **Abstract**

To provide a computer program, an extracorporeal circulation system, an observation device, and an information processing method that can prevent extracorporeal circulation from being performed in a state where a correspondence relationship between a position where pressure is measured and a pressure value is incorrect.

The computer program causes a computer to execute processing of acquiring a pressure value measured by each sensor from a plurality of the sensors that measure blood pressure at a plurality of positions in an extracorporeal circulation path that performs extracorporeal circulation of blood, and determining, on the basis of the acquired pressure value, whether or not the correspondence relationship between the pressure value and the position where the blood pressure is measured is correct.

## Description

### Technical Field

The present invention relates to a computer program that performs extracorporeal circulation of blood, an extracorporeal circulation system, an observation device, and an information processing method.

### Background Art

Conventionally, an extracorporeal circulation system that performs extracorporeal circulation of blood via an oxygenator that is present outside the body is used in some cases at the time of treatment of a person. The extracorporeal circulation is to circulate blood through a path that is present outside the body. A part of the blood circulation path is an extracorporeal circulation path that is present outside the body. The extracorporeal circulation path includes a pump that circulates blood and an oxygenator. The extracorporeal circulation system causes blood to flow out of a vein in the human body, exchanges gas in the blood by the oxygenator, and then returns the blood to a blood vessel (for example, an artery) in the human body. An example of the extracorporeal circulation system is an extracorporeal membrane oxygenation (ECMO). Patent Literature 1 discloses an example of the extracorporeal circulation system.

### Citation List

### Patent Literature

Patent Literature 1: JP 2019-217405 A

### Summary of Invention

### Technical Problem

At the time of performing the extracorporeal circulation, circulation of blood is controlled on the basis of various parameters of blood passing through the extracorporeal circulation path. The parameters include the pressure of blood. The pressure is one of the parameters necessary to understand the circulation state of blood. Usually, sensors for measuring the pressure are attached between the human body and the pump, between the pump and the oxygenator, and between the oxygenator and the human body. However, in a case where the correspondence relationship between a position where the pressure is measured and a pressure value is incorrect, a pressure value at an appropriate position becomes unknown, and thus, appropriate control is not performed, and there is a risk of causing a damage to the human body. For example, in a case where a cable for transmitting a measurement result from a sensor is connected to a device for observing the pressure, and another cable is incorrectly connected to a connection part to which a certain cable is to be connected, the correspondence relationship between the position where the pressure is measured and the pressure value becomes incorrect.

The present invention has been made in view of such circumstances, and an object of the present invention is to provide a computer program, an extracorporeal circulation system, an observation device, and an information processing method that can prevent extracorporeal circulation from being performed in a state where a correspondence relationship between a position where pressure is measured and a pressure value is incorrect.

### Solution to Problem

(1) A computer program according to one mode of the present invention causes a computer to execute processing of: acquiring, from a plurality of sensors that measure pressure of blood at a plurality of positions in an extracorporeal circulation path that performs extracorporeal circulation of blood, a pressure value measured by each of the sensors; and determining, on the basis of the pressure value that has been acquired, whether or not a correspondence relationship between the pressure value and the position where the pressure of blood is measured is correct.
(2) The computer program according to the above (1) preferably causes a computer to execute processing of: acquiring the pressure value from the plurality of sensors through a plurality of cables connected to the plurality of sensors and through a plurality of connection parts to which the plurality of cables are connected; having the correspondence relationship predetermined in accordance with which connection part through which the pressure value has been acquired; and determining whether or not the correspondence relationship that is predetermined is correct on the basis of the pressure value that has been acquired.
(3) The computer program according to the above (1) or (2) preferably causes a computer to execute processing of determining whether or not the correspondence relationship is correct while priming of the extracorporeal circulation path is being performed before the extracorporeal circulation is performed.
(4) The computer program according to any one of the above (1) to (3) preferably causes a computer to execute processing of determining whether or not the correspondence relationship is correct on the basis of whether the pressure value is a negative pressure value or a positive pressure value.
(5) The computer program according to any one of the above (1) to (4) preferably causes a computer to execute processing of determining whether or not the correspondence relationship is correct on the basis of a magnitude relationship among a plurality of the pressure values.
(6) The computer program according to any one of the above (1) to (5) preferably causes a computer to execute processing of, in a case where the pressure value associated with a first position between a pump and a human body included in the extracorporeal circulation path is not a negative pressure value, determining that a correspondence relationship between the first position and the pressure value is incorrect.
(7) The computer program according to any one of the above (1) to (6) preferably causes a computer to execute processing of, in a case where the pressure value associated with a second position between a pump and an oxygenator included in the extracorporeal circulation path or the pressure value associated with a third position between the oxygenator and a human body is not a positive pressure value, determining that a correspondence relationship between the second position and the pressure value, or a correspondence relationship between the third position and the pressure value is incorrect.
(8) The computer program according to the above (7) preferably causes a computer to execute processing of, in a case where the pressure value associated with the second position and the pressure value associated with the third position are positive pressure values and the pressure value associated with the second position is equal to or less than the pressure value associated with the third position, determining that a correspondence relationship between the second position and the pressure value, and a correspondence relationship between the third position and the pressure value are incorrect.
(9) The computer program according to any one of the above (1) to (8) further preferably causes a computer to execute processing of outputting a warning in a case where the correspondence relationship is incorrect.
(10) The computer program according to any one of the above (1) to (9) further preferably causes a computer to execute processing of outputting an instruction that appropriately associates the pressure value with a position where pressure of blood is measured in a case where the correspondence relationship is incorrect.
(11) The computer program according to any one of the above (1) to (10) further preferably causes a computer to execute processing of correcting the correspondence relationship in a case where the correspondence relationship is incorrect.
(12) An extracorporeal circulation system according to one mode of the present invention performs extracorporeal circulation of blood, the extracorporeal circulation system including: an extracorporeal circulation path that is disposed outside a human body and through which blood passes; a plurality of sensors that measure pressure of blood at a plurality of positions in the extracorporeal circulation path; and an observation device that observes pressure of blood, in which the observation device performs: acquiring pressure values measured by the plurality of sensors; and determining whether or not a correspondence relationship between the pressure value and a position where the pressure of blood is measured is correct on the basis of the pressure value that has been acquired.
(13) An observation device according to one mode of the present invention observes pressure of blood at a plurality of positions in an extracorporeal circulation path that performs extracorporeal circulation of blood, the observation device including: an acquisition unit that acquires, from a plurality of sensors that measure the pressure of blood at the plurality of positions, a pressure value measured by each of the sensors; and a determination unit that determines, on the basis of the pressure value that has been acquired, whether or not a correspondence relationship between the pressure value and the position where the pressure of blood is measured is correct.
(14) An information processing method according to one mode of the present invention includes: acquiring, from a plurality of sensors that measure pressure of blood at a plurality of positions in an extracorporeal circulation path that performs extracorporeal circulation of blood, a pressure value measured by each of the sensors; and
   determining, on the basis of the pressure value that has been acquired, whether or not a correspondence relationship between the pressure value and the position where the pressure of blood is measured is correct.

In one mode of the present invention, on the basis of the pressure values of the blood measured at a plurality of positions in the extracorporeal circulation path, it is determined whether or not the correspondence relationship between the position where the pressure of blood is measured and the pressure value is correct. The pressure values measured at the plurality of positions are values corresponding to the positions. Therefore, whether or not the correspondence relationship between the position where the pressure of blood is measured and the pressure value is correct can be determined on the basis of whether or not each pressure value is a value corresponding to the position. This enables the incorrectness in the correspondence relationship between the position and the pressure value to be found and the correspondence relationship to be corrected.

### Advantageous Effects of Invention

In the present invention, the blood extracorporeal circulation is prevented from being performed in a state in which the correspondence relationship between the pressure value and the position is incorrect. The present invention exhibits excellent effects such as that extracorporeal circulation of blood is appropriately performed and occurrence of a damage to the human body is prevented.

### Brief Description of Drawings

Fig. 1 is a schematic diagram illustrating a configuration example of an extracorporeal circulation system according to a first embodiment.
Fig. 2 is a block diagram illustrating an internal configuration example of a control device according to the first embodiment.
Fig. 3 is a schematic diagram illustrating a configuration of a portion of an interface unit.
Fig. 4 is a schematic graph illustrating a relationship between a pressure of blood and a position in an extracorporeal circulation path.
Fig. 5 is a schematic view illustrating an extracorporeal circulation path at the time when priming is performed.
Fig. 6 is a flowchart illustrating a procedure of a first example of processing for determining whether or not a correspondence relationship between a pressure value and a position performed by a control device is correct.
Fig. 7 is a flowchart illustrating a procedure of a second example of processing for determining whether or not a correspondence relationship between a pressure value and a position performed by a control device is correct.
Fig. 8 is a flowchart illustrating a procedure of the second example of processing for determining whether or not a correspondence relationship between a pressure value and a position performed by a control device is correct.
Fig. 9 is a flowchart illustrating a procedure of a third example of processing for determining whether or not a correspondence relationship between a pressure value and a position performed by a control device is correct.
Fig. 10 is a flowchart illustrating a procedure of the third example of processing for determining whether or not a correspondence relationship between a pressure value and a position performed by a control device is correct.
Fig. 11 is a schematic diagram illustrating a configuration example of an extracorporeal circulation system according to a second embodiment.
Fig. 12 is a block diagram illustrating an internal
configuration example of an observation device according to the second embodiment.

### Description of Embodiments

Hereinafter, the present invention will be specifically described with reference to the drawings illustrating embodiments of the present invention.

### <First Embodiment>

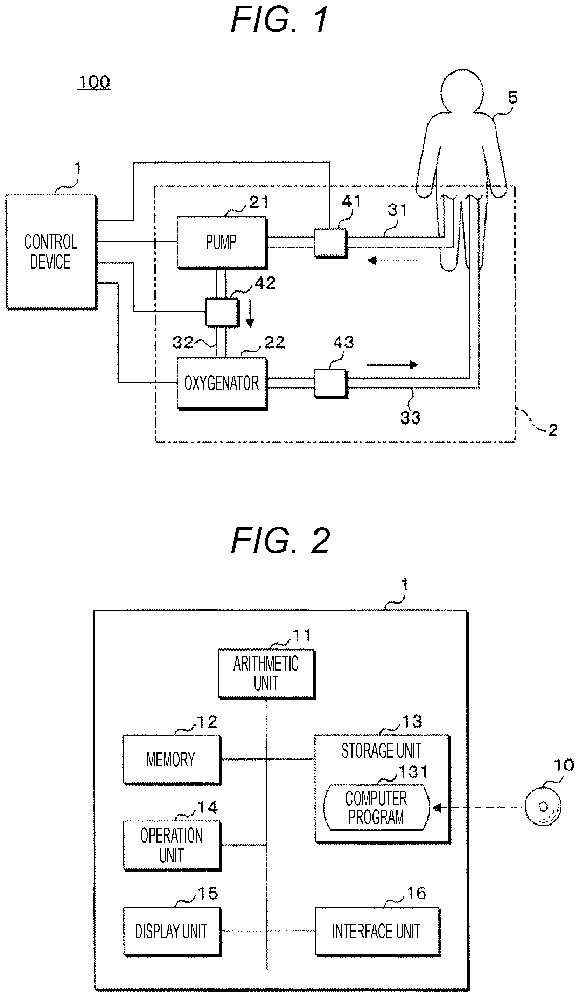

Fig. 1 is a schematic diagram illustrating a configuration example of an extracorporeal circulation system 100 according to a first embodiment. The extracorporeal circulation system 100 is a system that performs extracorporeal circulation of circulating blood of a human body 5 through an extracorporeal circulation path 2. The extracorporeal circulation path 2 is disposed outside the human body 5 and is a path through which the blood passes. The extracorporeal circulation path 2 includes a first blood supply tube 31, a second blood supply tube 32, a third blood supply tube 33, a pump 21, and an oxygenator 22. The first blood supply tube 31, the second blood supply tube 32, and the third blood supply tube 33 are bendable tubes and can allow the blood to pass therethrough. One end of the first blood supply tube 31 is connected to the pump 21, and the other end is connected to a vein of the human body 5. For example, the other end of the first blood supply tube 31 is connected to a catheter inserted into the vein of the human body 5. One end of the second blood supply tube 32 is connected to the pump 21, and the other end is connected to the oxygenator 22. One end of the third blood supply tube 33 is connected to the oxygenator 22, and the other end is connected to an artery of the human body 5. For example, the other end of the third blood supply tube 33 is connected to a catheter inserted into the artery of the human body 5. Note that the extracorporeal circulation path 2 is preferably a so-called closed system circuit that does not use a blood reservoir.

The pump 21 circulates the blood through the extracorporeal circulation path 2. By the operation of the pump 21, the blood is sucked out from the human body 5, and the blood flows through the first blood supply tube 31, the pump 21, the second blood supply tube 32, the oxygenator 22, and the third blood supply tube 33 in this order, and is returned into the human body 5. In Fig. 1, the direction in which the blood flows is indicated by an arrow. The oxygenator 22 takes in oxygen of gas blown into the oxygenator 22 and exchanges gas in the blood. The gas exchange removes carbon dioxide from the blood and adds oxygen to the blood. In this manner, the extracorporeal circulation system 100 assists the cardiopulmonary function of the human body 5.

The extracorporeal circulation system 100 includes a first sensor 41, a second sensor 42, and a third sensor 43 that measure the pressure of blood passing through the extracorporeal circulation path 2. The first sensor 41, the second sensor 42, and the third sensor 43 are provided at different positions in the extracorporeal circulation path 2, and measure the pressure of blood at a plurality of positions in the extracorporeal circulation path 2. The first sensor 41 is provided at a first position between the pump 21 and the human body 5. The first position may be in the middle of the first blood supply tube 31 or at an inlet of the pump 21. The second sensor 42 is provided at a second position between the pump 21 and the oxygenator 22. The second position may be at an outlet of the pump 21, in the middle of the second blood supply tube 32, or at an inlet of the oxygenator 22. The third sensor 43 is provided at a third position between the oxygenator 22 and the human body 5. The third position may be in the middle of the third blood supply tube 33 or at an outlet of the oxygenator 22.

The extracorporeal circulation system 100 includes a control device 1. The control device 1 is connected to the pump 21 and controls the operation of the pump 21. Each of the first sensor 41, the second sensor 42, and the third sensor 43 is connected to the control device 1 via a cable. The first sensor 41, the second sensor 42, and the third sensor 43 transmit sensor output values that change according to the pressure of blood to the control device 1 via a cable. The control device 1 receives the sensor output values transmitted from the first sensor 41, the second sensor 42, and the third sensor 43, and converts each of the received sensor output values into a measured pressure value of blood. In this manner, the control device 1 acquires the pressure values measured from the first sensor 41, the second sensor 42, and the third sensor 43. The control device 1 corresponds to an observation device and executes an information processing method.

Fig. 2 is a block diagram illustrating an internal configuration example of the control device 1 according to the first embodiment. The control device 1 is configured by using a computer. The control device 1 includes an arithmetic unit 11, a memory 12, a storage unit 13, an operation unit 14, a display unit 15, and an interface unit 16. The arithmetic unit 11 is constituted of, for example, a central processing unit (CPU), a graphics processing unit (GPU), or a multi-core CPU. The arithmetic unit 11 may be configured by using a quantum computer. The memory 12 stores temporary data generated in association with arithmetic operation. The memory 12 is, for example, a random access memory (RAM). The storage unit 13 is non-volatile and is, for example, a hard disk or a non-volatile semiconductor memory.

The storage unit 13 stores a computer program 131. For example, at the time of manufacturing the control device 1, the computer program 131 is read from a recording medium 10 such as an optical disk or a portable memory that stores the computer program 131 and is stored in the storage unit 13. The computer program 131 may be a computer program product. The computer program 131 may be downloaded from the outside and stored in the storage unit 13. The arithmetic unit 11 executes necessary processing according to the computer program 131.

The operation unit 14 receives the operation from a user. For example, the operation unit 14 is configured by using a push button or a touch panel. The display unit 15 displays information. The display unit 15 is, for example, a liquid crystal display or an electroluminescent display (EL display). The pump 21, the first sensor 41, the second sensor 42, and the third sensor 43 are connected to the interface unit 16. The arithmetic unit 11 controls the operation of the pump 21 and controls the extracorporeal circulation of blood by transmitting a control signal from the interface unit 16 to the pump 21. In addition, the control device 1 acquires a pressure value by receiving sensor output values transmitted from the first sensor 41, the second sensor 42, and the third sensor 43 by the interface unit 16 and converting each of the sensor output values into the pressure value of blood.

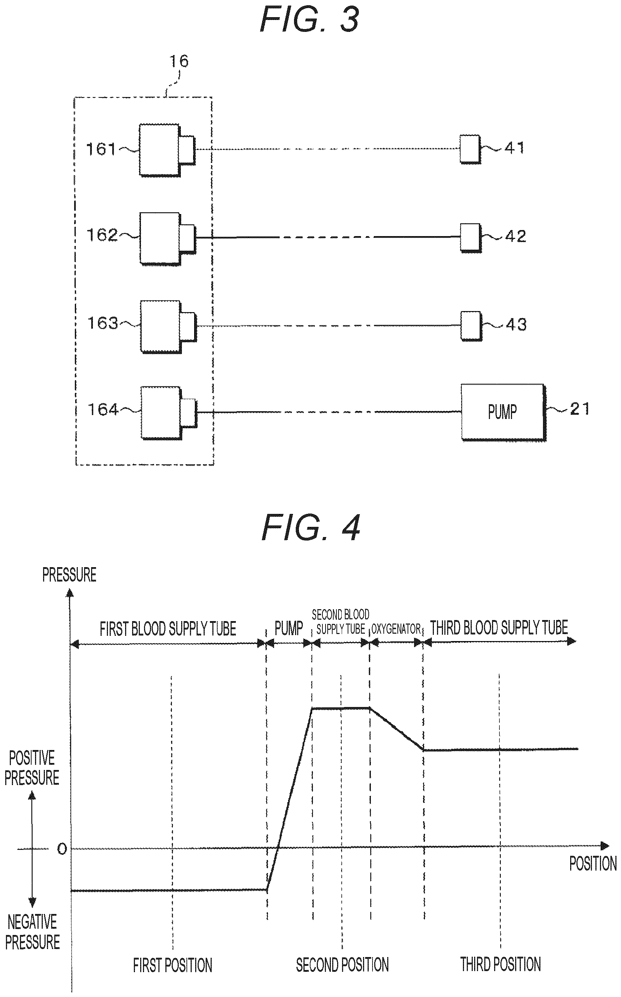

Fig. 3 is a schematic diagram illustrating a configuration of a portion of the interface unit 16. The interface unit 16 includes a first connection part 161 to which a cable connected to the first sensor 41 is connected, a second connection part 162 to which a cable connected to the second sensor 42 is connected, and a third connection part 163 to which a cable connected to the third sensor 43 is connected. The control device 1 receives sensor output values transmitted from the first sensor 41, the second sensor 42, and the third sensor 43 through the first connection part 161, the second connection part 162, the third connection part 163, and the cables connected to the respective connection parts. Hereinafter, the pressure value of blood converted from the sensor output value received by the control device 1 through the cable or the connection part is referred to as a pressure value acquired through the cable or the connection part. Furthermore, the interface unit 16 includes a fourth connection part 164 to which a cable connected to the pump 21 is connected. The control device 1 transmits a control signal to the pump 21 through the cable connected to the fourth connection part 164.

The control device 1 identifies the correspondence relationship between the pressure value acquired through the cable and the position where the pressure of blood is measured according to which one of the first connection part 161, the second connection part 162, and the third connection part 163 the cable is connected. Because the cable connected to the first sensor 41 provided at the first position is connected to the first connection part 161, the pressure value acquired through the first connection part 161 is associated with the first position. Similarly, the pressure value acquired through the second connection part 162 is associated with the second position, and the pressure value acquired through the third connection part 163 is associated with the third position. The control device 1 acquires the pressure values from the first sensor 41, the second sensor 42, and the third sensor 43, identifies at which position in the extracorporeal circulation path 2 each pressure value is measured according to which connection part the pressure value is acquired through, and controls the extracorporeal circulation of blood on the basis of the pressure value.

However, in a case where another cable is incorrectly connected to the connection part to which a certain cable is to be connected, the correspondence relationship between the position where the pressure is measured and the pressure value becomes incorrect. For example, in a case where the cable connected to the first sensor 41 is connected to the second connection part 162, the control device 1 incorrectly identifies the pressure value of the blood at the first position as the pressure value of the blood at the second position. At this time, the control device 1 or the user who operates the control device 1 executes improper control on the basis of an incorrect pressure value, and there is a risk of causing a damage such as hemolysis in the human body 5.

In order to prevent the inappropriate control, the control device 1 performs processing of determining whether or not the correspondence relationship between the acquired pressure value and the position where the pressure of blood is measured is correct before performing the extracorporeal circulation of blood. Determining whether or not the correspondence relationship between the acquired pressure value and the position where the pressure of blood is measured is correct is equivalent to determining whether or not the cable connected to each sensor is connected to the correct connection part.

The control device 1 determines whether or not the correspondence relationship between the position where the pressure of blood is measured and the pressure value is correct on the basis of the acquired pressure value. Fig. 4 is a schematic graph illustrating a relationship between a pressure of blood and a position in the extracorporeal circulation path 2. In Fig. 4, the horizontal axis represents the position in the extracorporeal circulation path 2, and the vertical axis represents the pressure of blood. Fig. 4 illustrates a relative pressure value based on the atmospheric pressure as the pressure value. In a case where the pressure of blood is above the atmospheric pressure, that is, in a case where the pressure value shown in Fig. 4 is positive, the pressure of blood is positive pressure. In a case where the pressure of blood is below the atmospheric pressure, that is, in a case where the pressure value shown in Fig. 4 is negative, the pressure of blood is negative pressure.

As illustrated in Fig. 4, at the time when the extracorporeal circulation of blood is performed, the pressure of blood is a pressure corresponding to the position in the extracorporeal circulation path 2. At a position between the pump 21 and the human body 5, the pressure of blood is a negative pressure. That is, the pressure value measured by the first sensor 41 provided at the first position is a negative pressure value.

The pump 21 increases the pressure of blood. At a position between the pump 21 and the oxygenator 22 and a position between the oxygenator 22 and the human body 5, the pressure of blood is the positive pressure. That is, the pressure value measured by the second sensor 42 provided at the second position and the pressure value measured by the third sensor 43 provided at the third position are positive pressure values. Furthermore, after passing through the oxygenator 22, the pressure of blood decreases. That is, the pressure value measured by the second sensor 42 provided at the second position is larger than the pressure value measured by the third sensor 43 provided at the third position.

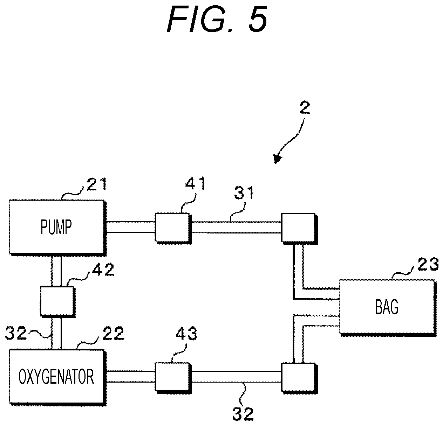

The extracorporeal circulation system 100 performs priming of the extracorporeal circulation path 2 before performing the extracorporeal circulation of blood. The priming is a treatment of filling the extracorporeal circulation path 2 with physiological saline and removing air bubbles from the inside of the extracorporeal circulation path 2. Fig. 5 is a schematic view illustrating the extracorporeal circulation path 2 at the time when the priming is performed. A bag 23 containing physiological saline is connected between the first blood supply tube 31 and the third blood supply tube 33. The pump 21 circulates the physiological saline through the extracorporeal circulation path 2. At this time, the control device 1 operates the pump 21 for a predetermined time, stops the pump 21 for a certain time, and controls the pump 21 to perform intermittent driving in which the operating and the stopping of the pump 21 are repeated. By the priming, the extracorporeal circulation path 2 is filled with the physiological saline, the air bubbles are removed from the inside of the extracorporeal circulation path 2, and preparation for the extracorporeal circulation of blood is made.

The extracorporeal circulation system 100 performs processing of determining whether or not the correspondence relationship between the acquired pressure value and the position where the pressure of blood is measured is correct while the priming of the extracorporeal circulation path 2 is being performed. By making the determination before the extracorporeal circulation of blood is performed, the determination can be made without putting strain on the human body 5. In addition, in a case where the correspondence relationship between the pressure value and the position is incorrect, the correspondence relationship can be corrected before the extracorporeal circulation of blood is performed, and the extracorporeal circulation of blood can be appropriately performed.

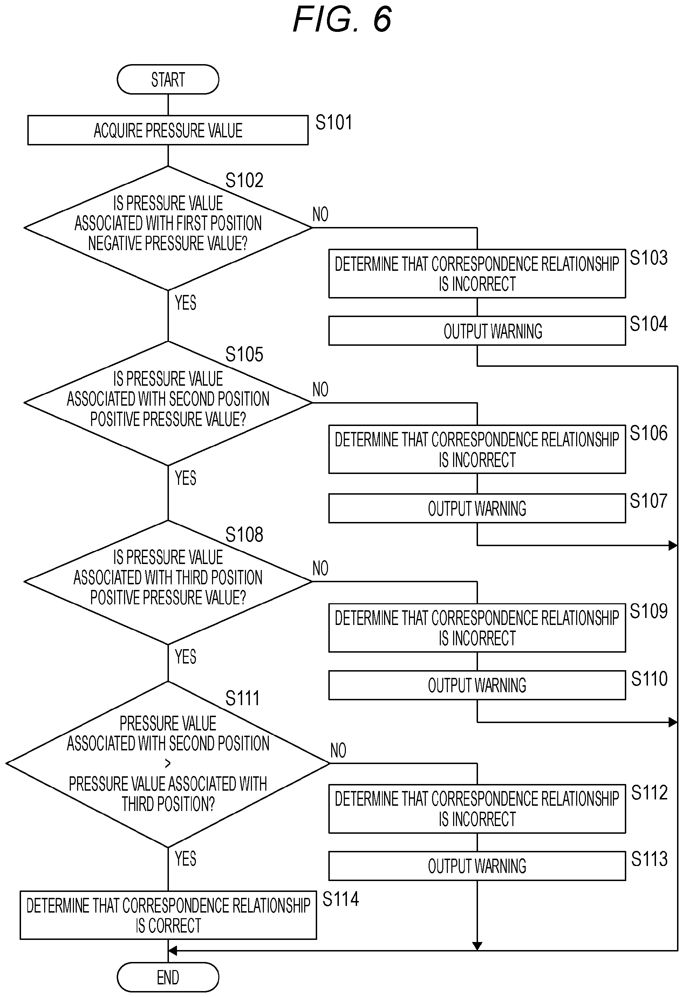

Fig. 6 is a flowchart illustrating a procedure of a first example of processing, performed by the control device 1, for determining whether or not the correspondence relationship between the pressure value and the position is correct. Hereinafter, step will be abbreviated as S. By the arithmetic unit 11 executing the information processing according to the computer program 131, the control device 1 executes the following processing. While the priming of the extracorporeal circulation path 2 is being performed, the first sensor 41, the second sensor 42, and the third sensor 43 measure the pressure of blood, and the control device 1 acquires the pressure values measured by the first sensor 41, the second sensor 42, and the third sensor 43 (S101). The first sensor 41, the second sensor 42, and the third sensor 43 transmit sensor output values that change according to the pressure of blood to the control device 1 via the cables. The control device 1 acquires the sensor output values through the first connection part 161, the second connection part 162, and the third connection part 163 to which the cables are connected. The arithmetic unit 11 converts the sensor output values into the pressure values of blood. In this manner, the control device 1 acquires the pressure value. The processing in S101 corresponds to an acquisition unit.

The control device 1 determines whether the pressure value associated with the first position is a negative pressure value (S102). The pressure value acquired through the first connection part 161 is associated with the first position. In S102, the arithmetic unit 11 determines whether or not the pressure value acquired through the first connection part 161 is a negative pressure value. If the pressure value is not the negative pressure value (S102: NO), the control device 1 determines that the correspondence relationship between the position where the pressure of blood is measured and the pressure value is incorrect (S103). In S103, the arithmetic unit 11 determines that the correspondence relationship between the pressure value acquired through the first connection part 161 and the first position is incorrect.

Next, the control device 1 outputs a warning indicating that the correspondence relationship between the position where the pressure of blood is measured and the pressure value is incorrect (S104). In S104, the arithmetic unit 11 outputs a warning by displaying a warning image in which contents of the warning are represented by an image on the display unit 15. For example, the arithmetic unit 11 displays, on the display unit 15, a warning image indicating that the connection of the cable to the first connection part 161 is incorrect. After S104 ends, the control device 1 ends the processing of determining whether or not the correspondence relationship is correct.

If the pressure value associated with the first position is the negative pressure value (S102: YES), the control device 1 determines whether or not the pressure value associated with the second position is a positive pressure value (S105). The pressure value acquired through the second connection part 162 is associated with the second position. In S105, the arithmetic unit 11 determines whether or not the pressure value acquired through the second connection part 162 is a positive pressure value. If the pressure value is not the positive pressure value (S105: NO), the control device 1 determines that the correspondence relationship between the position where the pressure of blood is measured and the pressure value is incorrect (S106). In S106, the arithmetic unit 11 determines that the correspondence relationship between the pressure value acquired through the second connection part 162 and the second position is incorrect.

Next, the control device 1 outputs a warning indicating that the correspondence relationship between the position where the pressure of blood is measured and the pressure value is incorrect (S107). In S107, the arithmetic unit 11 displays a warning image on the display unit 15. For example, the arithmetic unit 11 displays, on the display unit 15, a warning image indicating that the connection of the cable to the second connection part 162 is incorrect. After S107 ends, the control device 1 ends the processing of determining whether or not the correspondence relationship is correct.

If the pressure value associated with the second position is the positive pressure value (S105: YES), the control device 1 determines whether or not the pressure value associated with the third position is a positive pressure value (S108). The pressure value acquired through the third connection part 163 is associated with the third position. In S108, the arithmetic unit 11 determines whether or not the pressure value acquired through the third connection part 163 is a positive pressure value. If the pressure value is not the positive pressure value (S108: NO), the control device 1 determines that the correspondence relationship between the position where the pressure of blood is measured and the pressure value is incorrect (S109). In S109, the arithmetic unit 11 determines that the correspondence relationship between the pressure value acquired through the third connection part 163 and the third position is incorrect.

Next, the control device 1 outputs a warning indicating that the correspondence relationship between the position where the pressure of blood is measured and the pressure value is incorrect (S110). In S110, the arithmetic unit 11 displays a warning image on the display unit 15. For example, the arithmetic unit 11 displays, on the display unit 15, a warning image indicating that the connection of the cable to the third connection part 163 is incorrect. After S110 ends, the control device 1 ends the processing of determining whether or not the correspondence relationship is correct.

If the pressure value associated with the third position is the positive pressure value (S108: YES), the control device 1 determines whether or not the pressure value associated with the second position exceeds the pressure value associated with the third position (S111). In S111, the arithmetic unit 11 compares the pressure value acquired through the second connection part 162 with the pressure value acquired through the third connection part 163. If the pressure value associated with the second position is equal to or less than the pressure value associated with the third position (S111: NO), the control device 1 determines that the correspondence relationship between the position where the pressure of blood is measured and the pressure value is incorrect (S112). In S112, the arithmetic unit 11 determines that the correspondence relationship between the pressure value acquired through the second connection part 162 and the second position and the correspondence relationship between the pressure value acquired through the third connection part 163 and the third position are incorrect.

Next, the control device 1 outputs a warning indicating that the correspondence relationship between the position where the pressure of blood is measured and the pressure value is incorrect (S113). In S113, the arithmetic unit 11 displays a warning image on the display unit 15. For example, the arithmetic unit 11 displays, on the display unit 15, a warning image indicating that the connection of the cable to the second connection part 162 and the connection of the cable to the third connection part 163 are incorrect. After S113 ends, the control device 1 ends the processing of determining whether or not the correspondence relationship is correct.

If the pressure value associated with the second position exceeds the pressure value associated with the third position (S111: YES), the control device 1 determines that the correspondence relationship between the position where the pressure of blood is measured and the pressure value is correct (S114). The arithmetic unit 11 may display an image indicating that the correspondence relationship is correct on the display unit 15. The processing of S102, S103, S105, S106, S108, S109, S111, S112, and S114 corresponds to a determination unit. After S114 ends, the control device 1 ends the processing of determining whether or not the correspondence relationship is correct. Note that the control device 1 may include a sound output unit and output a warning by outputting a predetermined sound in S104, S107, S110, and S113. The control device 1 may omit the processing of S105 to S110.

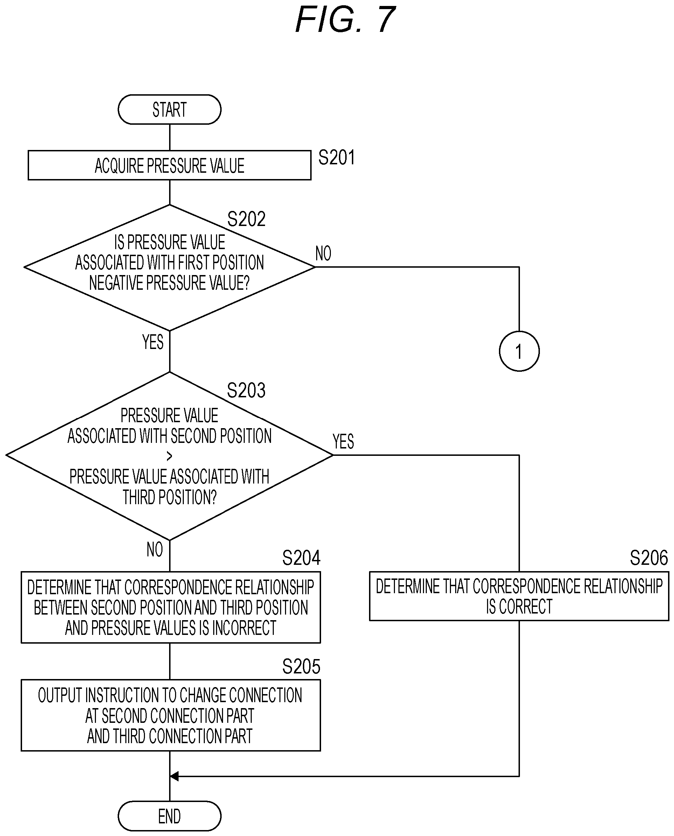

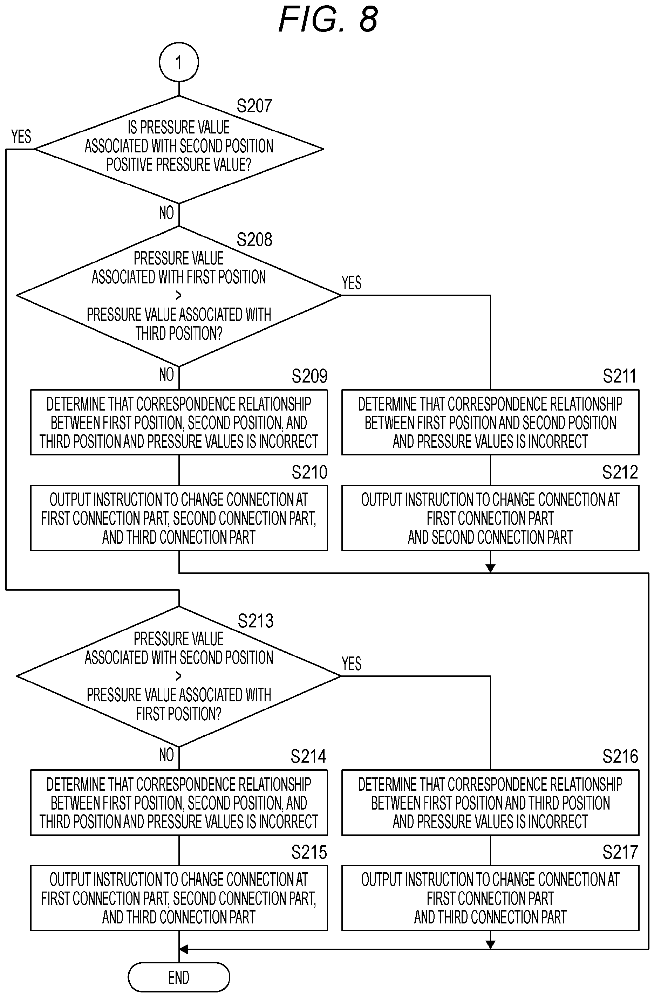

Figs. 7 and 8 are flowcharts illustrating a procedure of a second example of processing, performed by the control device 1, for determining whether or not the correspondence relationship between the pressure value and the position is correct. While the priming of the extracorporeal circulation path 2 is being performed, the control device 1 acquires the pressure values measured by the first sensor 41, the second sensor 42, and the third sensor 43 (S201). The processing in S201 corresponds to the acquisition unit. The control device 1 determines whether or not the pressure value associated with the first position is a negative pressure value (S202). In S202, the arithmetic unit 11 determines whether or not the pressure value acquired through the first connection part 161 is a negative pressure value.

If the pressure value is the negative pressure value (S202: YES), the control device 1 determines whether or not the pressure value associated with the second position exceeds the pressure value associated with the third position (S203). In S203, the arithmetic unit 11 compares the pressure value acquired through the second connection part 162 with the pressure value acquired through the third connection part 163. Because the pressure value associated with the first position is the negative pressure value, the pressure value associated with the second position and the pressure value associated with the third position are positive pressure values. Before S203, the control device 1 may determine whether or not the pressure value associated with the second position and the pressure value associated with the third position are positive pressure values.

If the pressure value associated with the second position is equal to or less than the pressure value associated with the third position (S203: NO), the control device 1 determines that the correspondence relationship between the second position and the third position and the pressure values is incorrect (S204). In S204, the arithmetic unit 11 determines that the correspondence relationship between the pressure value acquired through the second connection part 162 and the second position and the correspondence relationship between the pressure value acquired through the third connection part 163 and the third position are incorrect.

Next, the control device 1 outputs an instruction to change the connection at the second connection part 162 and the third connection part 163 (S205). In S205, the arithmetic unit 11 displays, on the display unit 15, an instruction image including an instruction to change the connection of the cables at the second connection part 162 and the third connection part 163. Because the correspondence relationship between the pressure value acquired through the second connection part 162 and the second position and the correspondence relationship between the pressure value acquired through the third connection part 163 and the third position are incorrect, the cables are incorrectly connected at the second connection part 162 and the third connection part 163. For example, a cable connected to the second sensor 42 provided at the second position is incorrectly connected to the third connection part 163, and a cable connected to the third sensor 43 provided at the third position is incorrectly connected to the second connection part 162.

By appropriately changing the connection of the cables at the second connection part 162 and the third connection part 163, the positions where the pressure of blood is measured and the pressure values are appropriately associated with each other. For example, by exchanging the cable connected to the second connection part 162 and the cable connected to the third connection part 163, the pressure values and the positions are appropriately associated with each other. In S205, the arithmetic unit 11 may display, on the display unit 15, an instruction image including an instruction to exchange the cable connected to the second connection part 162 and the cable connected to the third connection part 163. The pressure values and the positions are appropriately associated with each other by the user executing the work according to the output instruction. After S205 ends, the control device 1 ends the processing of determining whether or not the correspondence relationship is correct.

If the pressure value associated with the second position exceeds the pressure value associated with the third position (S203: YES), the control device 1 determines that the correspondence relationship between the position where the pressure of blood is measured and the pressure value is correct (S206). The arithmetic unit 11 may display an image indicating that the correspondence relationship is correct on the display unit 15. After S206 ends, the control device 1 ends the processing of determining whether or not the correspondence relationship is correct.

If the pressure value associated with the first position is not the negative pressure value (S202: NO), the control device 1 determines whether or not the pressure value associated with the second position is a positive pressure value (S207). In S207, the arithmetic unit 11 determines whether or not the pressure value acquired through the second connection part 162 is a positive pressure value.

If the pressure value associated with the second position is not the positive pressure value (S207: NO), the control device 1 determines whether or not the pressure value associated with the first position exceeds the pressure value associated with the third position (S208). In S208, the arithmetic unit 11 compares the pressure value acquired through the first connection part 161 with the pressure value acquired through the third connection part 163. Because the pressure value associated with the second position is not the positive pressure value, the pressure value associated with the third position is the positive pressure value. Because the pressure value associated with the first position is not the negative pressure value, the pressure value measured at the second position or the third position is associated with the first position. By comparing the pressure value associated with the first position with the pressure value associated with the third position, it becomes clear whether the pressure value associated with the first position is the pressure value measured at the second position or the third position.

If the pressure value associated with the first position is equal to or less than the pressure value associated with the third position (S208: NO), the control device 1 determines that the correspondence relationship between the first position, the second position, and the third position and the pressure values is incorrect (S209). In S209, the arithmetic unit 11 determines that the correspondence relationship between the pressure value acquired through the first connection part 161 and the first position, the correspondence relationship between the pressure value acquired through the second connection part 162 and the second position, and the correspondence relationship between the pressure value acquired through the third connection part 163 and the third position are incorrect. Specifically, it is determined that the pressure value acquired through the first connection part 161 is a pressure value measured at the third position, the pressure value acquired through the second connection part 162 is a pressure value measured at the first position, and the pressure value acquired through the third connection part 163 is a pressure value measured at the second position.

Next, the control device 1 outputs an instruction to change the connection at the first connection part 161, the second connection part 162, and the third connection part 163 (S210). In S210, the arithmetic unit 11 displays, on the display unit 15, an instruction image including an instruction to change the connection of the cables at the first connection part 161, the second connection part 162, and the third connection part 163. Because the correspondence relationship between the pressure values acquired through the first connection part 161, the second connection part 162, and the third connection part 163 and the positions where the pressure of blood is measured is all wrong, the connection of the cables in the first connection part 161, the second connection part 162, and the third connection part 163 is all wrong. Specifically, the cable connected to the first sensor 41 provided at the first position is incorrectly connected to the second connection part 162, the cable connected to the second sensor 42 provided at the second position is incorrectly connected to the third connection part 163, and the cable connected to the third sensor 43 provided at the third position is incorrectly connected to the first connection part 161.

By appropriately changing the connection of the cables at the first connection part 161, the second connection part 162, and the third connection part 163, the positions where the pressure of blood are measured and the pressure values are appropriately associated with each other. Specifically, by connecting the cable connected to the second connection part 162 to the first connection part 161, the cable connected to the third connection part 163 to the second connection part 162, and the cable connected to the first connection part 161 to the third connection part 163, the pressure values and the positions are appropriately associated with each other. In S210, the arithmetic unit 11 may display, on the display unit 15, an instruction image including an instruction to connect each cable to an appropriate connection part. The pressure values and the positions are appropriately associated with each other by the user executing the work according to the output instruction. After S210 ends, the control device 1 ends the processing of determining whether or not the correspondence relationship is correct.

If the pressure value associated with the first position exceeds the pressure value associated with the third position (S208: YES), the control device 1 determines that the correspondence relationship between the first position and the second position and the pressure values is incorrect (S211). Because the pressure value associated with the first position exceeds the pressure value associated with the third position, the pressure value associated with the first position is the pressure value measured at the second position, and the correspondence relationship between the third position and the pressure value is correct. In S211, the arithmetic unit 11 determines that the correspondence relationship between the pressure value acquired through the first connection part 161 and the first position and the correspondence relationship between the pressure value acquired through the second connection part 162 and the second position are incorrect. Specifically, it is determined that the pressure value acquired through the first connection part 161 is a pressure value measured at the second position, and the pressure value acquired through the second connection part 162 is a pressure value measured at the first position.

The control device 1 then outputs an instruction to change the connection at the first connection part 161 and the second connection part 162 (S212). In S212, the arithmetic unit 11 displays, on the display unit 15, an instruction image including an instruction to change the connection of the cables at the first connection part 161 and the second connection part 162. Because the correspondence relationship between the pressure values acquired through the first connection part 161 and the second connection part 162 and the positions where the pressure of blood are measured is incorrect, the connection of the cables at the first connection part 161 and the second connection part 162 is incorrect. Specifically, the cable connected to the first sensor 41 provided at the first position is incorrectly connected to the second connection part 162, and the cable connected to the second sensor 42 provided at the second position is incorrectly connected to the first connection part 161.

By appropriately changing the connection of the cables at the first connection part 161 and the second connection part 162, the positions where the pressure of blood is measured and the pressure values are appropriately associated with each other. For example, by exchanging the cable connected to the first connection part 161 and the cable connected to the second connection part 162, the pressure values and the positions are appropriately associated with each other. In S212, the arithmetic unit 11 may display, on the display unit 15, an instruction image including an instruction to exchange the cable connected to the first connection part 161 and the cable connected to the second connection part 162. The pressure values and the positions are appropriately associated with each other by the user executing the work according to the output instruction. After S212 ends, the control device 1 ends the processing of determining whether or not the correspondence relationship is correct.

If the pressure value associated with the second position is the positive pressure value (S207: YES), the control device 1 determines whether or not the pressure value associated with the second position exceeds the pressure value associated with the first position (S213). In S213, the arithmetic unit 11 compares the pressure value acquired through the first connection part 161 with the pressure value acquired through the second connection part 162. Because the pressure value associated with the first position is not the negative pressure value but the pressure value associated with the second position is the positive pressure value, the pressure value associated with the third position is the negative pressure value, and the pressure value measured at the first position is associated with the third position. One of the pressure values measured at the second position or the third position is associated with the first position, and the other is associated with the second position. By comparing the pressure value associated with the first position with the pressure value associated with the second position, it becomes clear whether the pressure value associated with the second position is the pressure value measured at the second position or the third position.

If the pressure value associated with the second position is equal to or less than the pressure value associated with the first position (S213: NO), the control device 1 determines that the correspondence relationship between the first position, the second position, and the third position and the pressure values is incorrect (S214). Specifically, in S214, the arithmetic unit 11 determines that the pressure value acquired through the first connection part 161 is a pressure value measured at the second position, the pressure value acquired through the second connection part 162 is a pressure value measured at the third position, and the pressure value acquired through the third connection part 163 is a pressure value measured at the first position.

Next, the control device 1 outputs an instruction to change the connection at the first connection part 161, the second connection part 162, and the third connection part 163 (S215). In S215, the arithmetic unit 11 displays, on the display unit 15, an instruction image including an instruction to change the connection of the cables at the first connection part 161, the second connection part 162, and the third connection part 163. In the first connection part 161, the second connection part 162, and the third connection part 163, all the cables are incorrectly connected. Specifically, the cable connected to the first sensor 41 provided at the first position is incorrectly connected to the third connection part 163, the cable connected to the second sensor 42 provided at the second position is incorrectly connected to the first connection part 161, and the cable connected to the third sensor 43 provided at the third position is incorrectly connected to the second connection part 162.

By appropriately changing the connection of the cables at the first connection part 161, the second connection part 162, and the third connection part 163, the positions where the pressure of blood are measured and the pressure values are appropriately associated with each other. Specifically, by connecting the cable connected to the third connection part 163 to the first connection part 161, the cable connected to the first connection part 161 to the second connection part 162, and the cable connected to the second connection part 162 to the third connection part 163, the pressure values and the positions are appropriately associated with each other. In S215, the arithmetic unit 11 may display, on the display unit 15, an instruction image including an instruction to connect each cable to an appropriate connection part. The pressure values and the positions are appropriately associated with each other by the user executing the work according to the output instruction. After S215 ends, the control device 1 ends the processing of determining whether or not the correspondence relationship is correct.

If the pressure value associated with the second position exceeds the pressure value associated with the first position (S213: YES), the control device 1 determines that the correspondence relationship between the first position and the third position and the pressure values is incorrect (S216). Because the pressure value associated with the second position exceeds the pressure value associated with the first position, the pressure value associated with the first position is the pressure value measured at the third position, and the correspondence relationship between the second position and the pressure value is correct. In S216, the arithmetic unit 11 determines that the correspondence relationship between the pressure value acquired through the first connection part 161 and the first position and the correspondence relationship between the pressure value acquired through the third connection part 163 and the third position are incorrect. Specifically, it is determined that the pressure value acquired through the first connection part 161 is a pressure value measured at the third position, and the pressure value acquired through the third connection part 163 is a pressure value measured at the first position.

Next, the control device 1 outputs an instruction to change the connection at the first connection part 161 and the third connection part 163 (S217). In S217, the arithmetic unit 11 displays, on the display unit 15, an instruction image including an instruction to change the connection of the cables at the first connection part 161 and the third connection part 163. Because the correspondence relationship between the pressure value acquired through the first connection part 161 and the first position and the correspondence relationship between the pressure value acquired through the third connection part 163 and the third position are incorrect, the cables are incorrectly connected at the first connection part 161 and the third connection part 163. Specifically, the cable connected to the first sensor 41 provided at the first position is incorrectly connected to the third connection part 163, and the cable connected to the third sensor 43 provided at the third position is incorrectly connected to the first connection part 161.

By appropriately changing the connection of the cables at the first connection part 161 and the third connection part 163, the positions where the pressure of blood is measured and the pressure values are appropriately associated with each other. For example, by exchanging the cable connected to the first connection part 161 and the cable connected to the third connection part 163, the pressure values and the positions are appropriately associated with each other. In S217, the arithmetic unit 11 may display, on the display unit 15, an instruction image including an instruction to exchange the cable connected to the first connection part 161 and the cable connected to the third connection part 163. The pressure values and the positions are appropriately associated with each other by the user executing the work according to the output instruction. After S217 ends, the control device 1 ends the processing of determining whether or not the correspondence relationship is correct. The processing of S202 to S204, S206, S207 to S209, S211, S213, S214, and S216 corresponds to the determination unit.

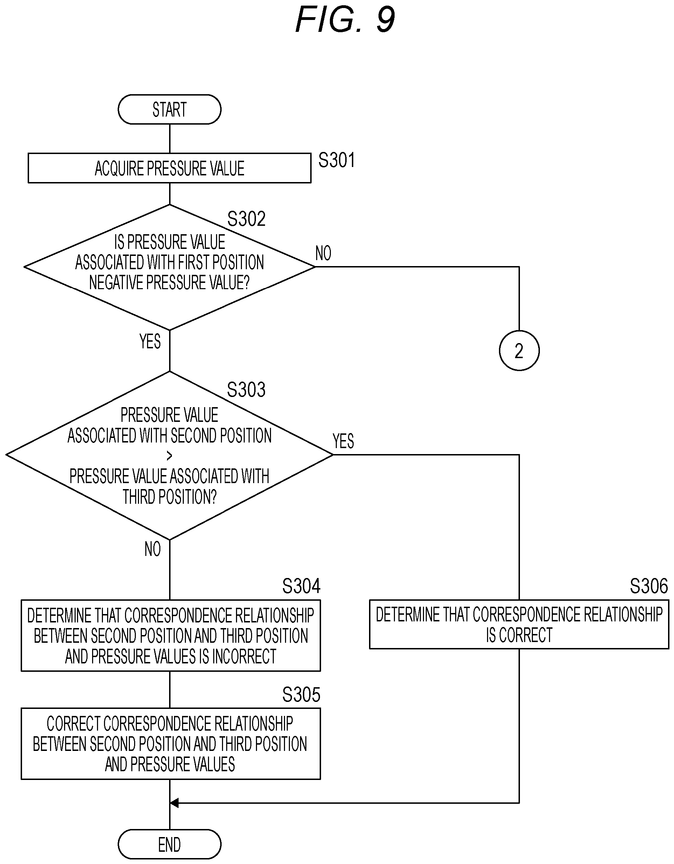

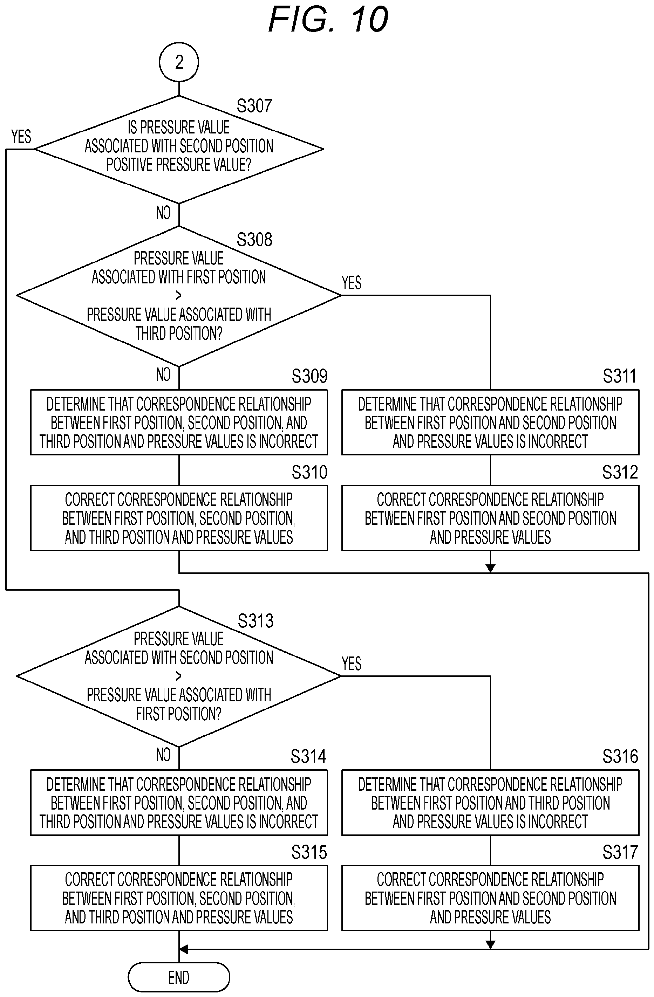

Figs. 9 and 10 are flowcharts illustrating a procedure of a third example of processing, performed by the control device 1, for determining whether or not the correspondence relationship between the pressure value and the position is correct. While the priming of the extracorporeal circulation path 2 is being performed, the control device 1 acquires the pressure values measured by the first sensor 41, the second sensor 42, and the third sensor 43 (S301). The processing in S301 corresponds to the acquisition unit. The control device 1 determines whether or not the pressure value associated with the first position is a negative pressure value (S302).

If the pressure value is the negative pressure value (S302: YES), the control device 1 determines whether or not the pressure value associated with the second position exceeds the pressure value associated with the third position (S303). Before S303, the control device 1 may determine whether or not the pressure value associated with the second position and the pressure value associated with the third position are positive pressure values.

If the pressure value associated with the second position is equal to or less than the pressure value associated with the third position (S303: NO), the control device 1 determines that the correspondence relationship between the second position and the third position and the pressure values is incorrect (S304). Next, the control device 1 corrects the correspondence relationship between the second position and the third position and the pressure values (S305). In S305, the arithmetic unit 11 corrects the correspondence relationship so as to cause the pressure value that has been associated with the second position to be associated with the third position, and cause the pressure value that has been associated with the third position to be associated with the second position. For example, the arithmetic unit 11 performs setting such that the pressure value acquired through the second connection part 162 is recognized as the pressure value measured by the third sensor 43, and the pressure value acquired through the third connection part 163 is recognized as the pressure value measured by the second sensor 42. For example, the arithmetic unit 11 stores information indicating the corrected correspondence relationship in the storage unit 13. After S305 ends, the control device 1 ends the processing of determining whether or not the correspondence relationship is correct.

If the pressure value associated with the second position exceeds the pressure value associated with the third position (S303: YES), the control device 1 determines that the correspondence relationship between the position where the pressure of blood is measured and the pressure value is correct (S306). The arithmetic unit 11 may display an image indicating that the correspondence relationship is correct on the display unit 15. After S306 ends, the control device 1 ends the processing of determining whether or not the correspondence relationship is correct.

If the pressure value associated with the first position is not the negative pressure value (S302: NO), the control device 1 determines whether or not the pressure value associated with the second position is a positive pressure value (S307). If the pressure value associated with the second position is not the positive pressure value (S307: NO), the control device 1 determines whether or not the pressure value associated with the first position exceeds the pressure value associated with the third position (S308).

If the pressure value associated with the first position is equal to or less than the pressure value associated with the third position (S308: NO), the control device 1 determines that the correspondence relationship between the first position, the second position, and the third position and the pressure values is incorrect (S309). Next, the control device 1 corrects the correspondence relationship between the first position, the second position, and the third position and the pressure values (S310). In S310, the arithmetic unit 11 corrects the correspondence relationship so as to cause the pressure value that has been associated with the second position to be associated with the first position, cause the pressure value that has been associated with the third position to be associated with the second position, and cause the pressure value that has been associated with the first position to be associated with the third position. For example, the arithmetic unit 11 performs setting such that the pressure value acquired through the second connection part 162 is recognized as the pressure value measured by the first sensor 41, the pressure value acquired through the third connection part 163 is recognized as the pressure value measured by the second sensor 42, and the pressure value acquired through the first connection part 161 is recognized as the pressure value measured by the third sensor 43. For example, the arithmetic unit 11 stores information indicating the corrected correspondence relationship in the storage unit 13. After S310 ends, the control device 1 ends the processing of determining whether or not the correspondence relationship is correct.

If the pressure value associated with the first position exceeds the pressure value associated with the third position (S308: YES), the control device 1 determines that the correspondence relationship between the first position and the second position and the pressure values is incorrect (S311). Next, the control device 1 corrects the correspondence relationship between the first position and the second position and the pressure values (S312). In S312, the arithmetic unit 11 corrects the correspondence relationship so as to cause the pressure value that has been associated with the first position to be associated with the second position, and cause the pressure value that has been associated with the second position to be associated with the first position. For example, the arithmetic unit 11 performs setting such that the pressure value acquired through the first connection part 161 is recognized as the pressure value measured by the second sensor 42, and the pressure value acquired through the second connection part 162 is recognized as the pressure value measured by the first sensor 41. For example, the arithmetic unit 11 stores information indicating the corrected correspondence relationship in the storage unit 13. After S312 ends, the control device 1 ends the processing of determining whether or not the correspondence relationship is correct.

If the pressure value associated with the second position is the positive pressure value (S307: YES), the control device 1 determines whether or not the pressure value associated with the second position exceeds the pressure value associated with the first position (S313). If the pressure value associated with the second position is equal to or less than the pressure value associated with the first position (S313: NO), the control device 1 determines that the correspondence relationship between the first position, the second position, and the third position and the pressure values is incorrect (S314).

Next, the control device 1 corrects the correspondence relationship between the first position, the second position, and the third position and the pressure values (S315). In S315, the arithmetic unit 11 corrects the correspondence relationship so as to cause the pressure value that has been associated with the third position to be associated with the first position, cause the pressure value that has been associated with the first position to be associated with the second position, and cause the pressure value that has been associated with the second position to be associated with the third position. For example, the arithmetic unit 11 performs setting such that the pressure value acquired through the third connection part 163 is recognized as the pressure value measured by the first sensor 41, the pressure value acquired through the first connection part 161 is recognized as the pressure value measured by the second sensor 42, and the pressure value acquired through the second connection part 162 is recognized as the pressure value measured by the third sensor 43. For example, the arithmetic unit 11 stores information indicating the corrected correspondence relationship in the storage unit 13. After S315 ends, the control device 1 ends the processing of determining whether or not the correspondence relationship is correct.

If the pressure value associated with the second position exceeds the pressure value associated with the first position (S313: YES), the control device 1 determines that the correspondence relationship between the first position and the third position and the pressure values is incorrect (S316). Next, the control device 1 corrects the correspondence relationship between the first position and the third position and the pressure values (S317). In S317, the arithmetic unit 11 corrects the correspondence relationship so as to cause the pressure value that has been associated with the first position to be associated with the third position, and cause the pressure value that has been associated with the third position to be associated with the first position. For example, the arithmetic unit 11 performs setting such that the pressure value acquired through the first connection part 161 is recognized as the pressure value measured by the third sensor 43, and the pressure value acquired through the third connection part 163 is recognized as the pressure value measured by the first sensor 41. For example, the arithmetic unit 11 stores information indicating the corrected correspondence relationship in the storage unit 13. After S317 ends, the control device 1 ends the processing of determining whether or not the correspondence relationship is correct. The processing of S302 to S304, S306, S307 to S309, S311, S313, S314, and S316 corresponds to the determination unit.

At the time of performing the processing of S305, S310, S312, S315, and S317, the control device 1 may perform processing of outputting that the correspondence relationship between the position where the pressure of blood is measured and the pressure value is corrected. For example, the arithmetic unit 11 displays an image indicating that the correspondence relationship has been corrected on the display unit 15. The control device 1 may perform processing of outputting a correction content of the correspondence relationship. For example, in S305, the arithmetic unit 11 displays, on the display unit 15, an image representing that the pressure value that has been associated with the second position is made to associate with the third position, and the pressure value that has been associated with the third position is made to associate with the second position. Similarly, in S310, the arithmetic unit 11 displays, on the display unit 15, an image representing that the pressure value that has been associated with the second position is made to associate with the first position, the pressure value that has been associated with the third position is made to associate with the second position, and the pressure value that has been associated with the first position is made to associate with the third position. In S312, S315, and S317, the arithmetic unit 11 performs processing in a similar manner.

As described above in detail, the extracorporeal circulation system 100 determines whether or not the correspondence relationship between the position where the pressure of blood is measured and the pressure value is correct on the basis of the pressure values of the blood measured at a plurality of positions in the extracorporeal circulation path 2. The pressure values measured at the plurality of positions are values corresponding to the positions. Whether or not the correspondence relationship between the position where the pressure of blood is measured and the pressure value is correct can be determined on the basis of whether or not each pressure value is a value corresponding to the position. The incorrectness in the correspondence relationship between the position and the pressure value can be found and the correspondence relationship can be corrected. Specifically, whether the pressure of the blood is positive pressure or negative pressure is determined by the position, and the magnitude of the two pressure values is determined by the positions where the respective pressure values are measured. Therefore, whether or not the correspondence relationship between the pressure value and the position is correct can be determined by determining whether the pressure value is a positive pressure value or a negative pressure value or by comparing the two pressure values.

In a mode in which a warning is output in a case where the correspondence relationship between the position where the pressure of blood is measured and the pressure value is incorrect, the position where the pressure of blood is measured and the pressure value are appropriately associated with each other by the user correctly reconnecting the cable connected to each sensor and the connection part according to the warning. In a mode in which an instruction for appropriately associating the pressure value and the position is output in a case where the correspondence relationship is incorrect, the pressure value and the position are appropriately associated with each other by the user executing work such as correctly reconnecting the cable and the connection part according to the output instruction. In a mode in which the correspondence relationship is corrected in a case where the correspondence relationship is incorrect, the pressure value and the position are appropriately associated with each other by correcting the correspondence relationship. Therefore, the blood extracorporeal circulation is prevented from being performed in a state in which the correspondence relationship between the pressure value and the position is incorrect.

The extracorporeal circulation system 100 performs the extracorporeal circulation of blood after the position where the pressure of blood is measured and the pressure value are appropriately associated with each other and the priming of the extracorporeal circulation path 2 is finished. The control device 1 acquires the pressure values measured by the first sensor 41, the second sensor 42, and the third sensor 43, and controls the extracorporeal circulation of blood on the basis of the acquired pressure values. Because the position where the pressure of blood is measured and the pressure value are appropriately associated with each other, the control device 1 can appropriately control the extracorporeal circulation of blood. Therefore, occurrence of a damage to the human body 5 due to improper control is prevented. In addition, at the time of preparing the extracorporeal circulation system 100 at the medical site, the user can make an appropriate preparation without requiring special attention to the correspondence relationship between the cable connected to each sensor and the connection part of the control device 1. Therefore, in an emergency, a burden on the user is reduced at a medical site full of tensed atmosphere.

In the first embodiment, the mode in which the three cables connected to the first sensor 41, the second sensor 42, and the third sensor 43 are connected to the control device 1 is shown, but the extracorporeal circulation system 100 may be configured such that the pressure value measured by each sensor is input to the control device 1 by another method. For example, the first sensor 41, the second sensor 42, and the third sensor 43 may be collectively connected to the control device 1 via one cable. Also in this mode, the control device 1 determines whether or not the correspondence relationship between the position where the pressure of blood is measured and the pressure value is correct by executing the processing of S101 to S114, S201 to S217, or S301 to S317. In S205, S208, and S211, the control device 1 outputs an instruction to correct the correspondence relationship between the position where the pressure of blood is measured and the pressure value. The user performs an operation for correcting the correspondence relationship on the operation unit 14, and the control device 1 performs the processing of correcting the correspondence relationship according to the operation. Also in this mode, the position where the pressure of blood is measured is appropriately associated with the pressure value, and the extracorporeal circulation of blood is appropriately controlled.

### <Second Embodiment>

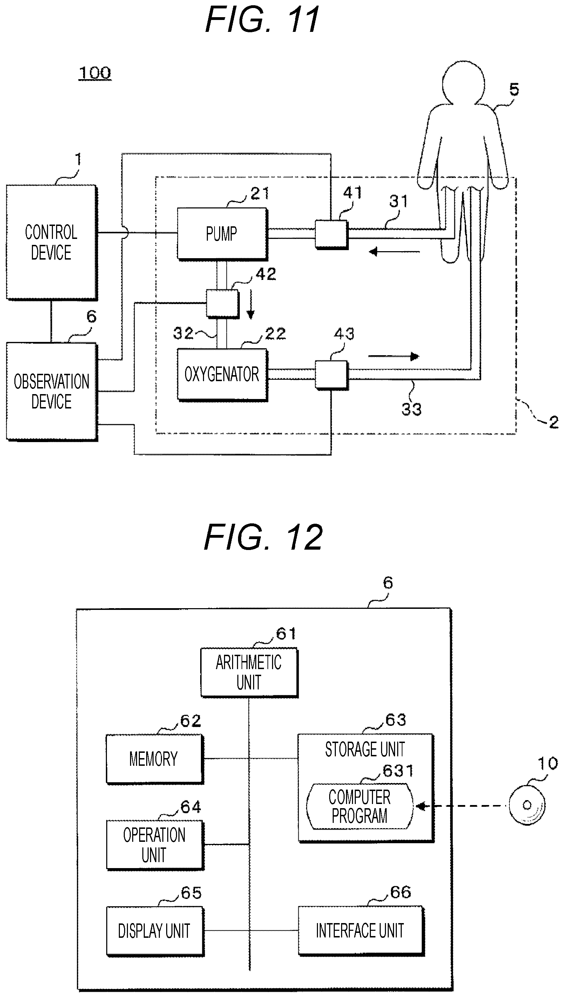

In the first embodiment, a mode in which the control device 1 corresponds to the observation device has been described, but in a second embodiment, a mode in which an extracorporeal circulation system 100 includes an observation device separately from a control device 1 is described. Fig. 11 is a schematic view illustrating a configuration example of the extracorporeal circulation system 100 according to the second embodiment. The extracorporeal circulation system 100 includes an observation device 6 that observes the pressure of blood at a plurality of positions in an extracorporeal circulation path 2. The observation device 6 is connected to the control device 1. In addition, cables connected to a first sensor 41, a second sensor 42, and a third sensor 43 are connected to the observation device 6. The configuration of the other portions of the extracorporeal circulation system 100 is similar to that of the first embodiment.

Fig. 12 is a block diagram illustrating an internal configuration example of the observation device 6 according to the second embodiment. The observation device 6 is configured by using a computer. The observation device 6 includes an arithmetic unit 61, a memory 62, a storage unit 63, an operation unit 64, a display unit 65, and an interface unit 66. The arithmetic unit 61 is configured by using, for example, a CPU, a GPU, or a multi-core CPU. The arithmetic unit 61 may be configured by using a quantum computer. The memory 62 stores temporary data generated in association with arithmetic operation. The memory 62 is, for example, a RAM. The storage unit 63 is non-volatile and is, for example, a hard disk or a non-volatile semiconductor memory.

The storage unit 63 stores a computer program 631. For example, at the time of manufacturing the observation device 6, the computer program 631 is read from a recording medium 60 such as an optical disk or a portable memory that stores the computer program 631 and is stored in the storage unit 63. The computer program 631 may be a computer program product. The computer program 631 may be downloaded from the outside and stored in the storage unit 63. The arithmetic unit 61 executes necessary processing according to the computer program 631.

The operation unit 64 receives the operation from a user. For example, the operation unit 64 is configured by using a push button or a touch panel. The display unit 65 displays information. The display unit 65 is, for example, a liquid crystal display or an **EL** display. The control device 1, the first sensor 41, the second sensor 42, and the third sensor 43 are connected to the interface unit 66. The interface unit 66 includes a plurality of connection parts to which a plurality of cables connected to the first sensor 41, the second sensor 42, and the third sensor 43 are connected. The observation device 6 acquires the pressure value by receiving the pressure values transmitted from the first sensor 41, the second sensor 42, and the third sensor 43 through the cables and the connection parts.

Similarly to the control device 1 in the first embodiment, the observation device 6 determines whether or not the correspondence relationship between the position where the pressure of blood is measured and the pressure value is correct by executing the processing of S101 to S114, S201 to S217, or S301 to S317. In S305, S310, S312, S315, and S317, the observation device 6 transmits an instruction to correct the correspondence relationship between the position where the pressure of blood is measured and the pressure value to the control device 1, and the control device 1 performs processing of correcting the correspondence relationship according to the instruction from the observation device 6.

As described above, in the second embodiment, the extracorporeal circulation system 100 determines whether or not the correspondence relationship between the position where the pressure of blood is measured and the pressure value is correct on the basis of the pressure values of the blood measured at the plurality of positions in the extracorporeal circulation path 2. Therefore, the position where the pressure of blood is measured is appropriately associated with the pressure value. The extracorporeal circulation system 100 performs the extracorporeal circulation of blood after the priming of the extracorporeal circulation path 2 is finished. The control device 1 acquires the pressure value via the observation device 6 and controls the extracorporeal circulation of blood on the basis of the acquired pressure value. The control device 1 may acquire the pressure values from the first sensor 41, the second sensor 42, and the third sensor 43 without passing through the observation device 6. Because the position where the pressure of blood is measured and the pressure value are appropriately associated with each other also in the second embodiment, the control device 1 can appropriately control the extracorporeal circulation of blood. Therefore, occurrence of a damage to the human body 5 due to improper control is prevented. In addition, similarly to the first embodiment, the burden on the user at the medical site is reduced.

In the second embodiment, the mode in which the three cables connected to the first sensor 41, the second sensor 42, and the third sensor 43 are connected to the observation device 6 is shown, but a mode in which the pressure value measured by each sensor is input to the observation device 6 by another method may be adopted. For example, the first sensor 41, the second sensor 42, and the third sensor 43 may be collectively connected to the observation device 6 via one cable. Also in this mode, the observation device 6 determines whether or not the correspondence relationship between the position where the pressure of blood is measured and the pressure value is correct by executing the processing of S101 to S114, S201 to S217, or S301 to S317. Also in this mode, the position where the pressure of blood is measured is appropriately associated with the pressure value, and the extracorporeal circulation of blood is appropriately controlled.

Some or all of the subject matters described in the respective embodiments can be combined together. In addition, some or all of the independent claims and their dependent claims described in the claims can be combined together, regardless of their dependent relationships. Furthermore, although a form (multiple dependent claim form) in which a claim dependent on two or more other claims is described is used in the claims, the claim form is not limited thereto. The present invention may be described by using a form in which a multiple dependent claim dependent on at least one multiple dependent claim is described.

The present invention is not limited to the content of the above-described embodiments, and various modifications can be made within the scope indicated in the claims. In other words, embodiments obtained by combining technical means appropriately changed within the scope indicated in the claims are also included in the technical scope of the present invention.

### Reference Signs List

- 100: Extracorporeal circulation system
- 1: Control device
- 131: Computer program
- 2: Extracorporeal circulation path
- 21: Pump
- 22: Oxygenator
- 31: First blood supply tube
- 32: Second blood supply tube
- 33: Third blood supply tube
- 41: First sensor
- 42: Second sensor
- 43: Third sensor
- 5: Human body
- 6: Observation device
- 631: Computer program

## Claims

1. A computer program causing a computer to execute processing of:
acquiring, from a plurality of sensors that measure pressure of blood at a plurality of positions in an extracorporeal circulation path that performs extracorporeal circulation of blood, a pressure value measured by each of the sensors; and
determining, on a basis of the pressure value that has been acquired, whether or not a correspondence relationship between the pressure value and the position where the pressure of blood is measured is correct.

2. The computer program according to claim 1, the computer program causing a computer to execute processing of:
acquiring the pressure value from the plurality of sensors through a plurality of cables connected to the plurality of sensors and through a plurality of connection parts to which the plurality of cables are connected;
having the correspondence relationship predetermined in accordance with which connection part through which the pressure value has been acquired; and
determining whether or not the correspondence relationship that is predetermined is correct on a basis of the pressure value that has been acquired.

3. The computer program according to claim 1 or 2, the computer program causing a computer to execute processing of
determining whether or not the correspondence relationship is correct while priming of the extracorporeal circulation path is being performed before the extracorporeal circulation is performed.

4. The computer program according to claim 1 or 2, the computer program causing a computer to execute processing of
determining whether or not the correspondence relationship is correct on a basis of whether the pressure value is a negative pressure value or a positive pressure value.

5. The computer program according to claim 1 or 2, the computer program causing a computer to execute processing of
determining whether or not the correspondence relationship is correct on a basis of a magnitude relationship among a plurality of the pressure values.

6. The computer program according to claim 1 or 2, the computer program causing a computer to execute processing of,
in a case where the pressure value associated with a first position between a pump and a human body included in the extracorporeal circulation path is not a negative pressure value, determining that a correspondence relationship between the first position and the pressure value is incorrect.

7. The computer program according to claim 1 or 2, the computer program causing a computer to execute processing of,
in a case where the pressure value associated with a second position between a pump and an oxygenator included in the extracorporeal circulation path or the pressure value associated with a third position between the oxygenator and a human body is not a positive pressure value, determining that a correspondence relationship between the second position and the pressure value, or a correspondence relationship between the third position and the pressure value is incorrect.

8. The computer program according to claim 7, the computer program causing a computer to execute processing of,
in a case where the pressure value associated with the second position and the pressure value associated with the third position are positive pressure values and the pressure value associated with the second position is equal to or less than the pressure value associated with the third position, determining that a correspondence relationship between the second position and the pressure value, and a correspondence relationship between the third position and the pressure value are incorrect.

9. The computer program according to claim 1 or 2, the computer program further causing a computer to execute processing of
outputting a warning in a case where the correspondence relationship is incorrect.

10. The computer program according to claim 1 or 2, the computer program further causing a computer to execute processing of
outputting an instruction that appropriately associates the pressure value with a position where pressure of blood is measured in a case where the correspondence relationship is incorrect.

11. The computer program according to claim 1 or 2, the computer program further causing a computer to execute processing of
correcting the correspondence relationship in a case where the correspondence relationship is incorrect.

12. An extracorporeal circulation system that performs extracorporeal circulation of blood, the extracorporeal circulation system comprising:
an extracorporeal circulation path that is disposed outside a human body and through which blood passes;
a plurality of sensors that measure pressure of blood at a plurality of positions in the extracorporeal circulation path; and
an observation device that observes pressure of blood,
wherein
the observation device performs:
acquiring pressure values measured by the plurality of sensors; and
determining whether or not a correspondence relationship between the pressure value and a position where the pressure of blood is measured is correct on a basis of the pressure value that has been acquired.

13. An observation device that observes pressure of blood at a plurality of positions in an extracorporeal circulation path that performs extracorporeal circulation of blood, the observation device comprising:
an acquisition unit that acquires, from a plurality of sensors that measure the pressure of blood at the plurality of positions, a pressure value measured by each of the sensors; and
a determination unit that determines, on a basis of the pressure value that has been acquired, whether or not a correspondence relationship between the pressure value and the position where the pressure of blood is measured is correct.

14. An information processing method comprising:
acquiring, from a plurality of sensors that measure pressure of blood at a plurality of positions in an extracorporeal circulation path that performs extracorporeal circulation of blood, a pressure value measured by each of the sensors; and
determining, on a basis of the pressure value that has been acquired, whether or not a correspondence relationship between the pressure value and the position where the pressure of blood is measured is correct.
